Europäisches Patentamt

European Patent Office

Office européen des brevets

(11) Publication number: **0 376 014**
**A1**

## EUROPEAN PATENT APPLICATION

(21) Application number: **89122587.2**

(51) Int. Cl.⁵: **A61B 1/24, A61C 5/02**

(22) Date of filing: **07.12.89**

(30) Priority: **14.12.88 JP 163003/88 U**
**25.08.89 JP 99261/89 U**

(43) Date of publication of application:
**04.07.90 Bulletin 90/27**

(84) Designated Contracting States:
**DE FR GB IT**

(71) Applicant: **Sugiyama, Masahiro**
**32-8, Kurobaru 2-chome Kokurakita-ku**
**Kitakyushu-shi Fukuoka 802(JP)**

(72) Inventor: **Sugiyama, Masahiro**
**32-8, Kurobaru 2-chome Kokurakita-ku**
**Kitakyushu-shi Fukuoka 802(JP)**

(74) Representative: **Tiedtke, Harro, Dipl.-Ing. et al**
**Patentanwaltsbüro Tiedtke-Bühling-Kinne-**
**Grupe-Pellmann-Grams-Struif Bavariaring 4**
**Postfach 20 24 03**
**D-8000 München 2(DE)**

(54) Dental instrument for preventing accidental swallow.

(57) A dental instrument (10) for preventing accidental swallow having a circumferential wall (14) for drop prevention on the periphery of a receiving plate (12) and a grip (13) on the receiving plate or circumferential wall; inserted in the mouth to catch the prosthetic appliance, filler, etc. coming off during a dental treatment so as to prevent them from dropping into the trachea or gullet.

Fig. 1

EP 0 376 014 A1

## DENTAL INSTRUMENT FOR PREVENTING ACCIDENTAL SWALLOW

Technical Field

The present invention relates to a dental instrument for preventing accidental swallow to be used in dental treatment.

Background Art

In dental treatment, it is necessary to attach or detach filler (filling material) or prosthetic appliance to the tooth or teeth to be treated, and the filler or prosthetic appliance may drop in the mouth,because they are attached or detached by hand. In such case, they can be sucked up by pump,etc. if they are small in size, but there sometimes has occurred an accident that the trachea is clogged by accidental swallow or aspiration.

There has also occurred another accident due to the reamer, etc. used in a treatment of root canal and dropped in the mouth during the treatment.

On the other hand, in dental treatment, dental mirror is used to examine the inside of the mouth, and this dental mirror consists of a small circular mirror to which a grip is attached for holding said mirror.

This dental mirror, however, is only for examing the inside of the mouth and not able to catch above-said filler, prosthetic appliance, etc.

Disclosure of Invention

The present invention has been made in view of above-mentioned circumstances and, accordingly, it is an object of this invention to provide a dental instrument for preventing accidental swallow which is able not only to serve as a dental mirror but to prevent an accidental swallow of filler, prosthetic appliance, etc.

The dental instrument for preventing accidental swallow relation to the first invention with the object mentioned above is a dental instrument comprising a receiving plate provided with a mirror on the top face and a grip attached to said receiving plate, characterized in that a circumferetial wall made of wire net for drop prevention is mounted on the periphery of above-said receiving plate.

The dental instrument for preventing accidental swallow relating to the second invention with the object mentioned above is a dental instrument comprising a receiving plate whose top face is a mirror formed by speculum treatment, characterized in that a circumferential wall made of wire net for drop prevention is mounted on the periphery of above-said receiving plate.

The dental instrument for preventing accidental swallow relating to the third invention with the object mentioned above is totally made of either metal or plastic material and characterized by comprising a receiving plate whose top face is a mirror, a plate-type circumferential wall formed as one piece on the periphery of said receiving plate, and a grip attached to above-said receiving plate.

And, the dental instrument for preventing accidental swallow relating to the fourth invention with the object mentioned above is totally made of either metal or plastic material and characterized by comprising a receiving plate whose top face is a mirror, a plate-type circumferential wall formed as one piece on the periphery of said receiving plate, and a grip attached to said circumferential wall.

In the case of dental instrument for preventing accidental swallow relating to the first, second, third, and fourth inventions, because there are provided a circumferential wall for drop prevention on the periphery of the receiving plate, and a grip on said receiving plate or circumferential wall, it becomes possible to set said dental instrument for preventing accidental swallow at the intended position in the mouth by holding said grip, and it is also possible to catch, for use of the receiving plate, the filler, prosthetic appliance, etc. for the tooth to be treated if they drop in the mouth, keeping them on the receiving plate by the circumferential wall provided on its periphery and,because there is provided a mirror on the top face of the receiving plate, it is also possible to examine the back side of the tooth by use of said mirror.

In the cases of above-said third and fourth inventions, it is also possible to provide draining holes at the bottom portion of the circumferential wall and, as a result, the saliva, etc. stay on the top surface of the receiving plate can be drained out.

Further,in the case of dental instrument for preventing accidental swallow relating to the first, second, third, and fourth inventions, it is also possible to provide a projecting setting jig on the top of the circumferential wall at the side opposite to the side at which the grip is attached.

In such case, the receiving plate can be settled at an intended position by putting said setting jig, for example, on the tooth of the lower jaw, and it becomes possible to catch, by use of the receiving plate and circumferential wall, the filler or, prosthetic appliance, etc. dropping toward the throat.

Brief Description of the Drawings

Fig.1 is a diagrammatic perspective view of a dental instrument for preventing accidental swallow relating to the first embodiment of the present invention;

Fig.2 is a sectional view, partially omitted, of said dental instrument for preventing accidental swallow;

Fig.3 is a diagrammatic side view of a dental instrument for preventing accidental swallow showing how it is used;

Fig.4 is a perspective view of a dental instrument for preventing accidental swallow relating to the second embodiment;

Fig.5 is a perspective view of a dental instrument for preventing accidental swallow relating to the third embodiment; and

Fig.6 is a diagrammatic perspective view of said dental instrument for preventing accidental swallow showing how it is used.

Best Mode for Carrying Out the Invention

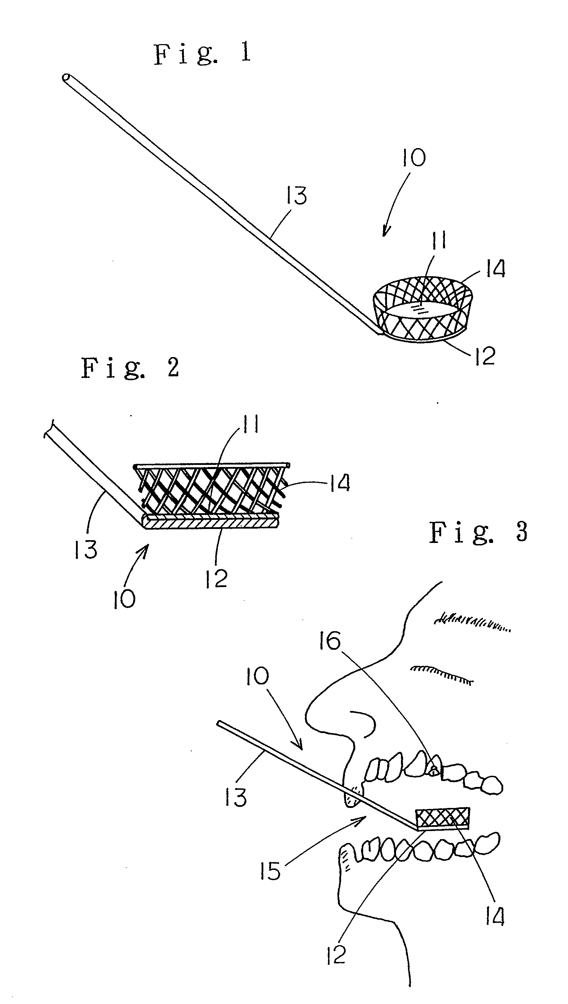

As shown in Figs.1 and 2, the dental instrument 10 for preventing accidental swallow relating to the first embodiment of the invention comprises a receiving plate 12 whose top face is provided with a mirror 11, a grip 13 which is attached to the one side of said receiving plate 12, and a circumferential wall 14 which is mounted on the periphery of above-said receiving plate 12. The details of these components will be described hereinunder.

The receiving plate 12, grip 13, and the circumferential wall 14 which are the components of said dental instrument 10 for preventing accidental swallow are made of corrosion-proof metal (such as stainless steel or brass) or plastic, and there is provided a separately-manufactured mirror 11 on the top face of the receiving plate as shown in Fig.2.

The diameter of above-said receiving plate 12 is approximately 10~ 30mm.

On the one side of said receiving plate 12, there is disposed an integrated- or attached-type grip 13. Though this grip 13 has a straight body in above-said embodiment, it is possible to provide concaves and convexes for slip prevention around the body and to form a hook or hole for hanging.

On the periphery of above-said receiving plate 12, there is mounted a circumferential wall 14 made of stainless steel wire net. Said circumferential wall 14 has 3 ~ 10mm height according to the diameter (10 ~ 30mm) of the receiving plate 12 and has such construction as to spread a little toward the top.

It is also possible to attach another circumferential wall made of different material such as transparent plastic, colored semitransparent plastic, or opaque plastic in place of above-said circumferential wall 14 made of wire net and, in such case, the circumferential wall may be of either net or plate.

Though the mirror 11 is separately manufactured and attached to the receiving plate 12 in this embodiment, it is possible to form a mirror on the top face of the receiving plate 12 by speculum treatment.

In actual use of said dental instrument 10 for preventing accidental swallow, it is set at the intended position in the mouth 15 with the grip 13 being held by hand as shown in Fig.3, and then a dental treatment is carried out. In this treatment, if the circumferential wall 14 made of wire net or transparent plastic is used, the back side of the tooth 16 to be treated can be examined in the mirror 11 through the circumferential wall 14 as well as examining directly in the mirror 11, because the wall has transparency.

And, if the filler, prosthetic appliance, etc. come off during treatment, they drop onto the receiving plate 12 and are caught there and taken out of the mouth as they are because there is provided a circumferential wall 14 for drop prevention on the periphery of the receiving plate 12.

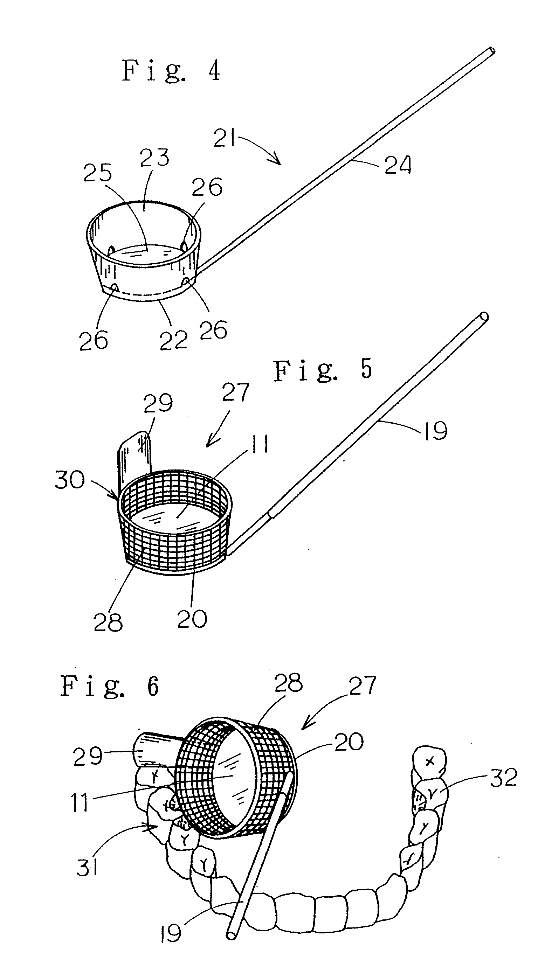

Then, the details will be described hereinunder on the dental instrument for preventing accidental swallow relating to the second and third embodiments shown in Figs. 4 through 6, and the same components are provided with the same number and the descriptions of these components are omitted.

Fig.4 shows the dental instrument 21 for preventing accidental swallow relating to the second embodiment of the invention and, in this case, the receiving plate 22, the circumferential wall 23, and the grip 24 are made of plastic (synthetic resin), and there is formed a mirror 25 on the top surface of the receiving plate 22 by speculum treatment. And, this enables an easy manufacture of said dental instrument 21 for preventing accidental swallow at low cost. And, since the circumferential wall is made of plate-like material in this dental instrument 21 for preventing accidental swallow, there are provided draining holes 26 at the bottom portion of the circumferential wall 23 so as to drain saliva, etc. staying in the instrument.

In addition, though the body of dental instrument 21 for preventing accidental swallow is totally made of plastic, it is possible to manufacture the instrument with metal.

Figs.5 and 6 show the dental instrument 27 for preventing accidental swallow relating to the third embodiment of the invention and, as shown in the figures, there is provided a setting jig 29 on the top section of the circumferential wall 28.

This setting jig 29 is attached on the top section of the circumferential wall 28 (having 3 ~

10mm height) which is mounted on the periphery of the receiving plate 20.

This setting jig 29 is made of corrosion-resistant metal or plastic and attached with clockwise or counterclockwise drift from the just opposite side 30 of the grip 19.

Accordingly, in the use of this dental instrument 27 for preventing accidental swallow as shown in Fig.6, the dental instrument 27 for preventing accidental swallow is put into the mouth at the intended position with the grip 19 being held by hand and, in this case, either one side of abovesaid setting jig is contacted to the top face of the tooth 31 on the lower jaw, and then the treatment of the tooth 31 is carried out. And, if the filler, prosthetic appliance, etc. come off during the treatment, they drop onto the circumferential wall 28 or the receiving plate 20 on the surface of which a mirror 11 is formed and are taken out of the mouth as they are. Then, an accidental swallow can be prevented.

In Fig.6, said dental instrument 27 for preventing accidental swallow is set to the left side tooth 31 and, in the case of treatment on the right side tooth 32, the setting jig 29 is positioned symmetrically to the position mentioned in the fourth embodiment based on above-said just opposite side 30 of the grip 19.

In addition, in the case of above-said embodiment, it is possible to prevent an accidental swallow of filler, prosthetic appliance, etc. during the treatment for both right and left teeth on the lower jaw by use of only one dental instrument for preventing accidental swallow having a swivel grip which can be turned at the connection to the receiving plate.

Industrial Applicability

By applying above-said first, second, third and fourth inventions, it is possible to prevent an accidental swallow of filler, prosthetic appliance, etc. used during a dental treatment. And, because of the receiving plate having a mirror on its top face, this dental instrument can be used for examing the back side of the tooth in the mouth, as well as serving as a receiver for preventing accidental swallow.

And, by providing aforesaid setting jig to the circumferential wall of the dental instrument for preventing accidental swallow relating to the first, second, third and fourth embodiments of the invention, and by contacting the the setting jig to the tooth of lower jaw, it is possible to prevent an accidental swallow of filler, prosthetic appliance, etc. which may occur during the dental treatment of said tooth.

A dental instrument for preventing accidental swallow having a circumferential wall for drop prevention on the periphery of a receiving plate and a grip on the receiving plate or circumferential wall; inserted in the mouth to catch the prosthetic appliance, filler, etc. coming off during a dental treatment so as to prevent them from dropping into the trachea or gullet.

Claims

1. A dental instrument for preventing accidental swallow comprising a receiving plate provided with a mirror on the top face and a grip attached to said receiving plate, characterized in that a circumferential wall made of wire net for drop prevention is mounted on the periphery of above-said receiving plate.

2. A dental instrument for preventing accidental swallow comprising a receiving plate whose top face is a mirror formed by speculum treatment, characterized in that a circumferential wall made of wire net for drop prevention is mounted on the periphery of above-said receiving plate.

3. A dental instrument for preventing accidental swallow totally made of either metal or plastic material, characterized by comprising a receiving plate whose top face is a mirror, a plate-type circumferential wall formed as one piece on the periphery of said receiving plate, and a grip attached to above-said receiving plate.

4. A dental instrument for preventing accidental swallow totally made of either metal or plastic material, characterized by comprising a receiving plate whose top face is a mirror, a plate-type circumferential wall formed as one piece on the periphery of said receiving plate, and a grip attached to said circumferential wall.

5. A dental instrument for preventing accidental swallow set forth in claim 3 or 4, wherein draining holes are formed at the bottom portion of the circumferential wall.

6. A dental instrument for preventing accidental swallow set forth in claim 1, 2, 3 or 4, wherein a projecting setting jig is provided on the top of the circumferential wall at the side opposite to the side at which the grip is attached.

Fig. 1

Fig. 2

Fig. 3

Fig. 4

Fig. 5

Fig. 6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB-A-1 440 561  (AB CERCO)<br>* Description figures *<br>--- | 1-6 | A 61 B    1/24<br>A 61 C    5/02 |
| X | US-A-1 345 718  (CHESTER)<br>* Page 1, lines 71-97; figures 1,5 *<br>----- | 1-6 | |
| | | | TECHNICAL FIELDS SEARCHED (Int. Cl.5)<br><br>A 61 B<br>A 61 C |

The present search report has been drawn up for all claims

| Place of search | Date of completion of the search | Examiner |
|---|---|---|
| THE HAGUE | 25-03-1990 | VANRUNXT J.M.A. |

EPO FORM 1503 03.82 (P0401)